# EUROPEAN PATENT APPLICATION

(11) **EP 4 111 975 A1**
(43) Date of publication of application: **04.01.2023**
(21) Application number: 21207978.4
(22) Date of filing: 12.11.2021
(51) Int. Cl.: A61B 5/28, A61B 5/00

(54) **MRI COMPATIBLE NODE-BASED ECG MEASUREMENT NETWORK**

(30) Priority: 30.06.2021 US 202163216886 P
(71) Applicant: Koninklijke Philips N.V., 5656 AG Eindhoven (NL)
(72) Inventor: APPLETON, Bruce Geoffrey, Eindhoven (NL); REDDER, Paul Franz, Eindhoven (NL)
(74) Representative: Philips Intellectual Property & Standards

(57) **Abstract**

An ECG measurement node for a patient electrode is provided, including an electrode interface and ECG processing circuitry electrically coupled to the electrode interface. The electrode interface is electrically and removably coupled to the patient electrode. The patient electrode is affixed to a patient. The ECG processing circuitry is configured to generate an ECG signal. The ECG measurement node further includes a transceiver configured to wirelessly transmit the ECG signal or a fiber optic interface configured to transmit the ECG signal via a fiber optic link. An ECG measurement network is also provided, including a first and second ECG measurement node. The first ECG measurement node and the second ECG measurement node are communicatively coupled to a patient monitor. The ECG measurement network may further include a central access point communicatively coupled to the first ECG measurement node, the second ECG measurement node, and the patient monitor.

## Description

### FIELD OF THE DISCLOSURE

The present disclosure is directed generally to systems and methods for measuring electrocardiogram (ECG) signals in, for example, magnetic resonant imaging (MRI) applications.

### BACKGROUND

Magnetic resonant imaging (MRI) scanners utilize magnetic fields, magnetic field gradients, and radio waves to generate a series of images of a patient. In order to focus on the relevant images, the MRI may use a gating or trigger-mechanism. One such mechanism can be an electrocardiogram (ECG).

An ECG measurement typically requires affixing one or more electrodes to the patient. These electrodes then typically connect to a patient monitor via a series of lead wires. However, use of physical lead wires creates a number of complications. First, during the operation of the MRI scanner, the lead wires are highly susceptible to picking up MRI noise. The MRI noise can lead to signal-to-noise degradation of the ECG signal, and other undesired effects. The degradation of the ECG signal can reduce the accuracy of any gating or triggering based on the degraded ECG signal.

Further, a lead wire of length in an MRI scanner may unintentionally act as a radio frequency (RF) antenna. By acting as an RF antenna, the lead wire can pick up RF energy generated by the MRI scanner. This RF energy can cause RF heating of the lead wire, potentially heating the wire to the point of burning the patient undergoing the MRI scan.

Additionally, wired, galvanic connections between the electrodes and a mains network require safety protections for the patient in case of mains network malfunction.

Even further, the lead wires can significantly restrict patient movement following electrode placement. If the patient needs to be re-positioned during the MRI scan, the patient may require assistance from a technician to avoid tangling the lead wires or displacing the electrodes.

Accordingly, there is a need in the art for improved systems for capturing ECG signals in an MRI environment.

### SUMMARY OF THE DISCLOSURE

The present disclosure is generally directed to an electrocardiogram (ECG) measurement node and a distributed ECG measurement network of ECG measurement nodes. The ECG measurement nodes are configured to convey ECG signals to a patient monitor without lead wires, thus reducing signal degradation and safety issues associated with lead wires in a magnetic resonant imaging (MRI) environment. The ECG measurement node removably couples to a patient electrode affixed to the skin of a patient undergoing an MRI scan. The ECG measurement node captures the electrical signal generated by the patient electrode, and generates an ECG signal based on the captured electrical signal via ECG processing circuitry.

The ECG measurement node leadlessly and non-galvanically conveys the ECG signal to other devices, such as a patient monitor, a central access point, or one or more additional ECG measurement nodes. In one example, the ECG measurement node includes a transceiver to wirelessly transmit the ECG signal. In an alternate example, the ECG measurement node includes a fiber optic interface. The fiber optic interface is configured to optically transmit the ECG signal via a fiber optic link. The transmission of the ECG signals via wireless transceiver or fiber optic link is supported by the ECG processing circuitry of the ECG measurement node.

The ECG measurement node can also receive or transmit control signals. The control signals can dictate various aspects of the processing circuitry of the ECG measurement node. The control signals can also control various aspects of the central access point, the patient monitor, and/or additional ECG measurement nodes.

Multiple ECG measurement nodes can be used to form an ECG measurement network. In this way, an ECG measurement network can generate ECG signals from multiple ECG measurement nodes affixed to a patient, and convey the ECG signals to a patient monitor for analysis. In one example, each ECG measurement node is directly connected to the patient monitor, either by wireless connection or fiber optic link. In a further example, a central access node may function as an intermediary between one or more of the ECG measurement nodes and patient monitor. In an even further example, the one or more ECG measurement nodes and/or a central access point can form a mesh network to convey the ECG signals to the patient monitor in a dynamic, non-hierarchical manner.

Generally, in one aspect, an electrocardiogram (ECG) measurement node for a patient electrode is provided. The ECG measurement node includes an electrode interface. The electrode interface is electrically and removably coupled to the patient electrode. According to an example, the patient electrode is affixed to a patient.

The ECG measurement node further includes ECG processing circuitry. The ECG processing circuitry is electrically coupled to the electrode interface. The ECG processing circuitry is configured to generate an ECG signal. According to an example, the ECG processing circuitry includes one or more amplifiers, one or more filters, and/or one or more analog-to-digital converters.

According to an example, the ECG measurement node further includes a battery.

According to an example, the ECG measurement node further includes a transceiver configured to wirelessly transmit the ECG signal.

According to an example, the ECG measurement node further includes a fiber optic interface configured to transmit the ECG signal via a fiber optic link.

Generally, in another aspect, an ECG measurement network is provided. The ECG measurement network includes a first ECG measurement node for a first patient electrode. The first ECG measurement node includes a first electrode interface electrically and removably coupled to the first patient electrode. The first ECG measurement node further includes first ECG processing circuitry electrically coupled to the first electrode interface. The first ECG processing circuitry is configured to generate a first ECG signal.

The ECG measurement network further includes a second ECG measurement node for a second patient electrode. The second ECG measurement node includes a second electrode interface electrically and removably coupled to the second patient electrode. The second ECG measurement node further includes second ECG processing circuitry electrically coupled to the second electrode interface. The second ECG processing circuitry is configured to generate a second ECG signal. The first ECG measurement node and the second ECG measurement node are communicatively coupled to a patient monitor. According to an example, the first ECG measurement node is wirelessly coupled to the patient monitor.

According to an example, the ECG measurement network, further includes a central access point communicatively coupled to the first ECG measurement node, the second ECG measurement node, and the patient monitor. The central access point may be wirelessly coupled to the patient monitor. The first ECG measurement node may be communicatively coupled to the central access point via a fiber optic link.

According to an example, the first ECG measurement node is communicatively coupled to the second ECG measurement node. The first ECG measurement node may be wirelessly coupled to the second ECG measurement node. The first ECG measurement node may be communicatively coupled to the second ECG measurement node via a central access point. The first ECG measurement node and the second ECG measurement node may form a local mesh network.

In various implementations, a processor or controller may be associated with one or more storage media (generically referred to herein as "memory," e.g., volatile and non-volatile computer memory such as RAM, PROM, EPROM, EEPROM, floppy disks, compact disks, optical disks, magnetic tape, SSD, etc.). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects as discussed herein. The terms "program" or "computer program" are used herein in a generic sense to refer to any type of computer code (e.g., software or microcode) that can be employed to program one or more processors or controllers.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is an illustration of an electrocardiogram (ECG) measurement node, in accordance with an example.
FIG. 2 is a schematic drawing of an ECG measurement node, in accordance with an example.
FIG. 3 is a schematic drawing of an ECG measurement node, in accordance with a further example.
FIG. 4 is a schematic drawing of a wireless ECG measurement network, in accordance with an example.
FIG. 5 is a schematic drawing of a fiber optic ECG measurement network, in accordance with an example.
FIG. 6 is a schematic drawing of a hybrid ECG measurement network, in accordance with an example.

### DETAILED DESCRIPTION OF EMBODIMENTS

The present disclosure is generally directed to an electrocardiogram (ECG) measurement node and a distributed ECG measurement network of ECG measurement nodes. The ECG measurement nodes are configured to convey ECG signals to a patient monitor without lead wires, thus reducing signal degradation and safety issues associated with lead wires in a magnetic resonant imaging (MRI) environment.

The ECG measurement node removably couples to a patient electrode affixed to the skin of a patient undergoing an MRI scan. The ECG measurement node captures the electrical signal generated by the patient electrode, and generates an ECG signal based on the captured electrical signal via ECG processing circuitry. The ECG processing circuitry can be configured to amplify and/or filter the electrical signal captured by the patient electrode. Further, the ECG processing circuitry can also include an analog-to-digital converter (ADC) to convert the analog electrical signal captured by the patient electrode to a digital signal. This ECG processing circuitry, as well as other aspects of the ECG measurement node, can be powered by a battery within the housing of the node.

The ECG measurement node leadlessly conveys the ECG signal to other devices, such as a patient monitor, a central access point, or one or more additional ECG measurement nodes. In one example, the ECG measurement node includes a transceiver to wirelessly transmit the ECG signal. In an alternate example, the ECG measurement node includes a fiber optic interface configures to optically transmit the ECG signal via a fiber optic link. The transmission of the ECG signals via wireless transceiver or fiber optic link is supported by the ECG processing circuitry of the ECG measurement node. In traditional ECG configurations, the functions of the ECG processing circuitry are performed at the patient monitor. By distributing the processing circuitry to the various measurement points, the ECG signal may be amplified, filtered, digitized, or otherwise processed to enable wireless or optical communication. Further, in both the wireless and fiber optic configurations, the ECG measurement node is galvanically isolated from the receiving device, thus reducing signal degradation and safety issues associated with lead wires in an MRI environment.

Multiple ECG measurement nodes can be used to form an ECG measurement network. In this way, an ECG measurement network can generate ECG signals from multiple ECG measurement nodes affixed to a patient, and convey the ECG signals to a patient monitor for analysis. In one example, each ECG measurement node is directly connected to the patient monitor, either by wireless connection or fiber optic link. In a further example, a central access node may function as an intermediary between one or more of the ECG measurement nodes and patient monitor. In an even further example, the one or more ECG measurement nodes and/or a central access point can form a mesh network to convey the ECG signals to the patient monitor in a dynamic, non-hierarchical manner. The patient monitor and/or the central access point can also be utilized to synchronize the ECG signals, ensuring more accurate analysis.

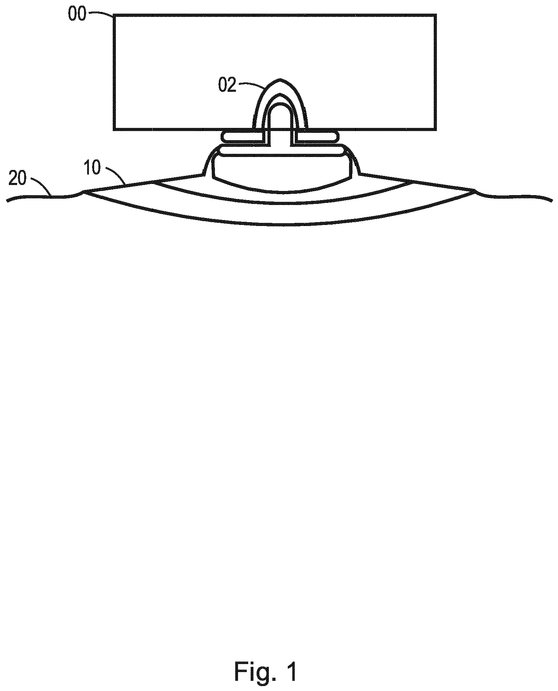

FIG. 1 shows an example ECG measurement node 100 for a patient electrode 10. As can be seen in FIG. 1, patient electrode 10 is affixed to the skin of a patient 20. In traditional ECG arrangements, the patient electrode 10 would transfer electric signals generated by the body of the patient to a lead wire. The lead wire would then convey this electric signal to a patient monitor for analysis. However, in an MRI environment, these lead wires are susceptible to signal degradation as well as safety concerns (such as burning) due to radio frequency (RF) heating of the lead wires. The present invention overcomes these issues with an ECG measurement node 100 to capture the electric signals from the body of the patient 20, and then convey an ECG signal 106 corresponding to the captured electric signal to a patient monitor 30. The ECG measurement node 100 mechanically and electrically couples to the patient electrode 10 via an electrode interface 102. In a preferred example, the electrode interface 102 is removably coupled to the patient electrode 10. In alternative examples, the electrode interface 102 may be permanently mechanically coupled to the patient electrode 10.

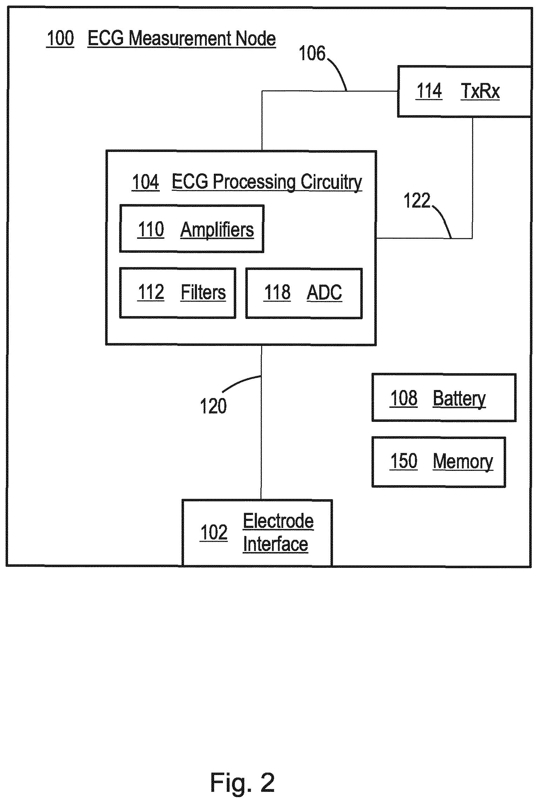

FIG. 2 shows a schematic diagram of an example ECG measurement node 100 configured to wirelessly transmit an ECG signal 106. As illustrated in FIG. 1, the electrode interface 102 electrically couples to the patient electrode 10. The electrode interface 102 conveys the electrode signal 120 from the patient electrode 10 to ECG processing circuitry 104. The ECG processing circuitry 104 processes the electrode signal 120 into an ECG signal 106 to be transmitted to a patient monitor 30 for analysis, display, and/or further processing. The ECG processing circuitry 104 can contain one or more amplifiers 110 to increase the amplitude of the electrode signal 120. The ECG processing circuitry 104 can also contain one or more filters 112 to remove undesired frequency aspects from the electrode signal 120. The ECG processing circuitry 104 can also contain one or more ADCs 112 to digitize the electrode signal 120 prior to transmission. The ECG processing circuitry 104 can further include any other circuits and/or circuitry to prepare the ECG signal 106 for transmission.

The ECG signal 106 generated by the ECG processing circuitry 104 is then provided to the wireless transceiver 114. The wireless transceiver 114 is configured to modulate the ECG signal 106 to a carrier signal for wireless transmission. The wireless transceiver 114 may be an RF transceiver configured to transmit the ECG signal 106 in the RF spectrum. The transceiver 144 may be further configured to transmit the ECG signal 106 according to a number of protocols, such as Bluetooth, Zigbee, Wi-Fi, etc. The transmitted ECG signal 106 may be configured to be received by a patient monitor 30, a central access point 40, and/or a second ECG measurement node 100b.

The wireless transceiver 114 can also be configured to receive wireless signals from other devices. For example, the ECG measurement node 100 can be arranged in a local mesh network 250 with a second ECG measurement node 100b. The second ECG measurement node 100b may transmit a second ECG signal 106b to the first ECG measurement node 100. The ECG measurement node 100 may then forward the second ECG signal 106b on to the patient monitor 30, the central access point 40, and/or a third ECG measurement node 100c. The ECG measurement node 100 may also store the second ECG signal in memory 150.

In a further example, the wireless transceiver 114 can also be configured to receive a control signal 122 from the patient monitor 30 or central access point 40. The transceiver can convey this control signal 122 to the ECG processing circuitry 104. The control signal 122 can dictate various aspects of the ECG processing circuitry 104. In one example, the control signal 122 controls the timing aspects of the ECG processing circuitry 104 ensure the ECG signal 106 is synchronized in the time domain with at least a second ECG signal 106b generated by a second ECG measurement node 106b.

The various components of the ECG measurement node 100 may be powered by a battery 108. The battery 108 may be of any type suitable to power the components of the ECG measurement node 100, while also of suitable size to fit within the housing of the ECG measurement node 100. In further examples, the housing and components of the ECG measurement node 100 are composed of non-ferrous metals, as the magnetic fields generated by the MRI scanner can cause ferrous metals to be pulled violently towards the magnetic source.

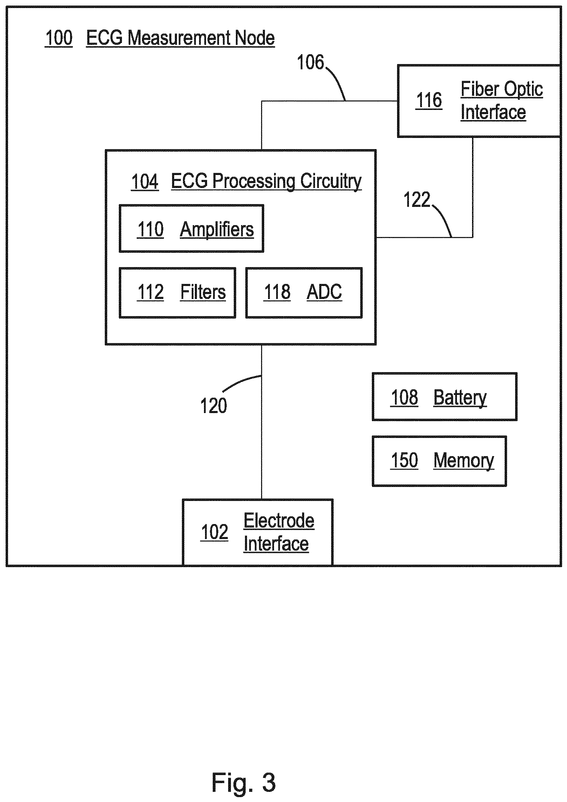

FIG. 3 shows an ECG measurement node 100 similar to that of FIG. 2. However, in FIG. 3, rather than a wireless transceiver 114, the ECG measurement node includes a fiber optic interface 116. The fiber optic interface 116 is configured to optically transmit the ECG signal 106 via a fiber optic link 210 connected to the fiber optic interface 116. By modulating the ECG signal 106 onto an optical carrier signal, the fiber optic interface 116 may transmit the ECG signal 106 to a patient monitor 30, a central access point 40, and/or a second ECG measurement node 100b connected to the fiber optic link 210. Further, modulating the ECG signal 106 onto the optical carrier signal enables the ECG measurement node 100 to be galvanically isolated from the destination device.

In a further example, and similar to the wireless transceiver 114 of the ECG measurement node of FIG. 2, the fiber optic interface 116 and the fiber optic link 210 may enable bidirectional communication between the ECG measurement node 100 and a patient monitor 30, a central access point 40, and/or a second ECG measurement node 100b. In this way, the ECG measurement node 100 may receive a second ECG measurement signal 106b from another device. Further, the ECG measurement node 100 may receive a control signal 122 as described above.

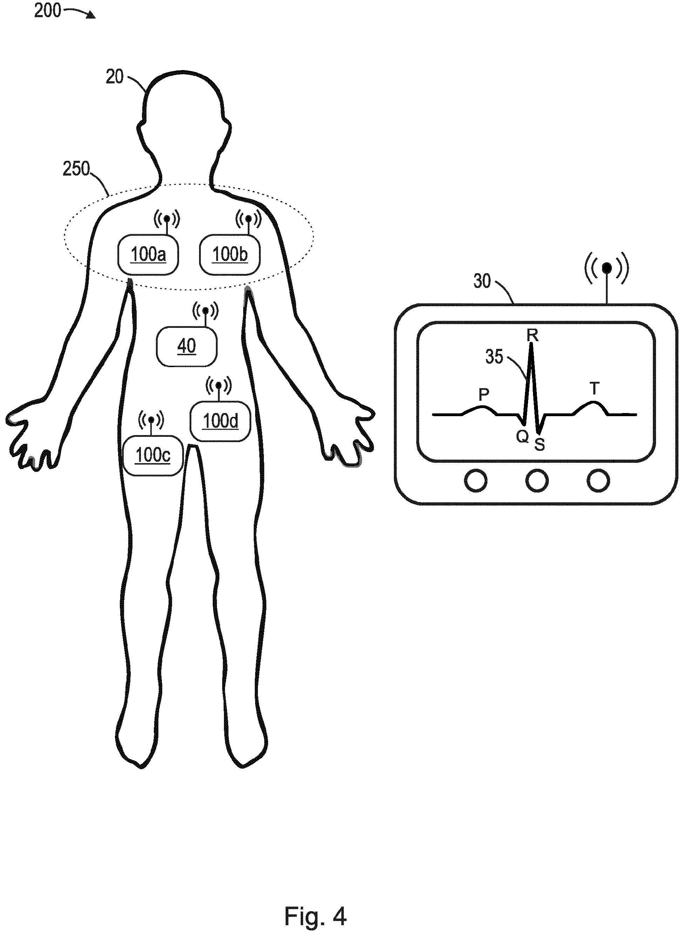

FIG. 4 shows a distributed ECG measurement network 200 collecting ECG signals 106 from a patient 20. The patient 20 may be undergoing an MRI scan, and the ECG signals 106 may be used to gate and/or trigger the data collected by the MRI scan.

The ECG measurement network 200 includes four ECG measurement nodes 100a-100d placed at various positions on the patient 20. Each of the ECG measurement nodes 100a-100d includes a wireless transceiver 114 as shown in FIG. 2. The ECG measurement network 200 further includes a central access point 40 and a patient monitor 30.

According to one example, each ECG measurement node 100a-100d wirelessly communicates directly with the patient monitor 30. In this example, the patient monitor 30 receives ECG signals 106a-106d wirelessly transmitted by the wireless transceiver 114a-1 14d of each ECG measurement node 100a-100d. The patient monitor 30 then processes the received ECG signals 106a-106d to generate and display an ECG waveform 35. The ECG waveform 35 may represent the voltage potential between two or more ECG signals 106a-106d.

According to another example, one ECG measurement node 100a wirelessly transmits an ECG signal 106a to a central access point 40. Upon receiving the ECG signal 106a, the central access point 40 is configured to transmit the ECG signal 106a to the patient monitor 30. In this way, the central access point 40 acts as an intermediary between the ECG measurement node 100a and the patient monitor 30. The central access point 40 can include a transceiver, memory, processor, and any other circuitry required to receive and receive ECG signals 106. In one example, the ECG measurement node 100a may not be able to produce a strong enough wireless carrier to convey the ECG signal 106 to the patient monitor 30. This may be due to hardware limitations of the ECG processing circuitry 104a or the position of the ECG measurement node 100a relative to the patient monitor 30. In this example, the central access point 40 is able to receive the ECG signal 106, even if the patient monitor 30 cannot. The central access point 40 may then transmit the ECG signal 106 to the patient monitor 30.

In a further example, each ECG measurement node 100a-100d transmits their associated ECG signal 106a-106d to the central access point 40. The central access point 40 subsequently transmits each ECG signal 106a-106b to the patient monitor 30.

In a further example, the central access point 40 may be able to transmit information to one of the ECG measurement nodes 100a. For example, the central access point 40 may transmit a control signal 122a to the ECG measurement node 100a. This control signal 122a may be generated by the patient monitor 30, and relayed to the ECG measurement node 100a via the central access point 40. This control signal 122a can be used by the ECG measurement nodes 100a-100d to synchronize their generated ECG signals 106a-106d to ensure accurate analysis by the patient monitor 30.

In a further example, ECG measurement nodes 100a and 100b may be communicatively coupled such that they form a local mesh network 250. A local mesh network 250 allows the ECG measurement nodes 100a-100d to convey their associated ECG signals 106a-106b to the patient monitor 30 in a dynamic, non-hierarchical manner. For example, rather than transmitting ECG signal 106a directly to the central access point 40 or the patient monitor 30, a first ECG measurement node 100a may transmit a first ECG signal 106a to a second ECG measurement node 100b. The second ECG measurement node 100b may then transmit the first ECG signal 106a to either the central access point or the patient monitor 30. The local mesh network 250 may contain any number of ECG measurement nodes 100 in any number of combinations of links in order to optimize the conveyance of ECG signal 106. Further, the ECG measurement network 200 may include multiple local mesh networks 250 to convey ECG signals 106 to the central access point 40 or the patient monitor 30.

Similarly, the local mesh network 250 allows the patient monitor 30 to convey control signals 122 to the ECG measurement nodes 100. For example, rather than transmitting control signal 122a directly to a first ECG measurement node 100a, the patient monitor may first transmit the control signal to a second ECG measurement node 100b, either directly or via central access point 40. The second ECG measurement node 100b may then transmit the control signal 122a to the first ECG measurement node 100a.

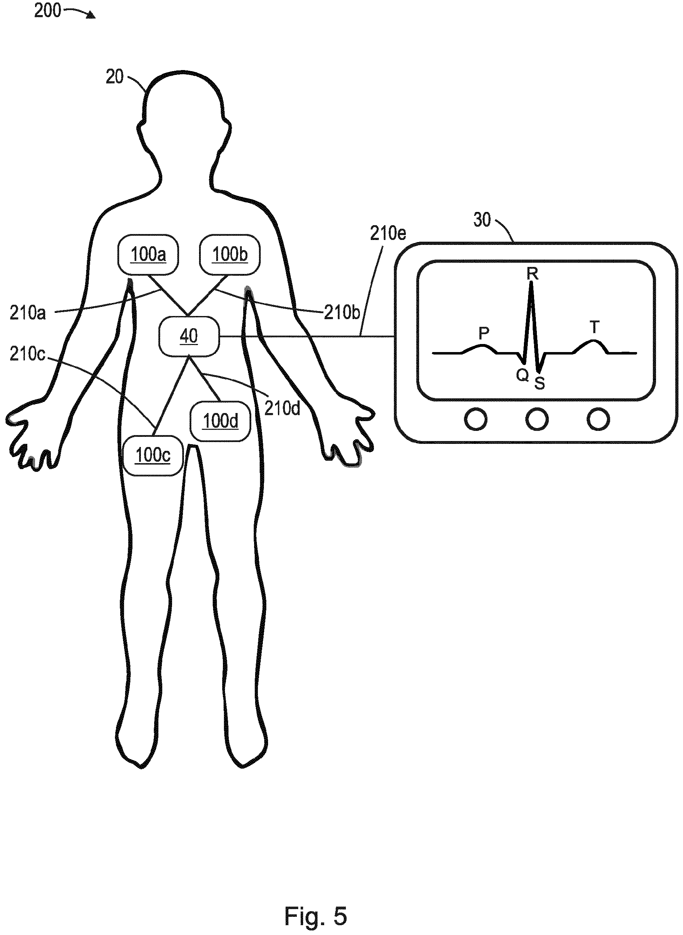

FIG. 5 shows an ECG measurement network 200 similar to that of FIG. 4. However, in FIG. 5, rather communicating wirelessly, the ECG measurement nodes 100a-100d are each communicatively coupled to the central access point 40 via fiber optic links 210a-210d. The central access point 40 is then communicatively coupled to the patient monitor 30 via fiber optic link 210e. In this example, the central access point 40 includes four fiber optic interfaces to connect to the fiber optic links 210a-210d. Further, by modulating the ECG signal 106 onto an optical carrier signal, each of the ECG measurement nodes 100a-100d are galvanically isolated from the central access point 40.

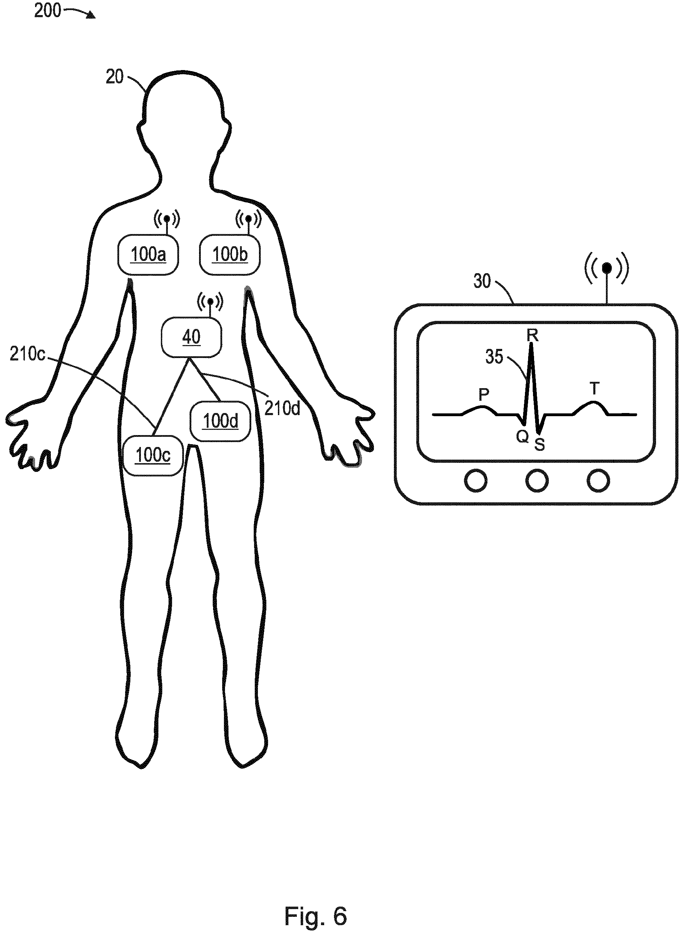

FIG. 6 shows an ECG measurement network 200 which is a hybrid of FIG. 4 and FIG. 5. In the ECG measurement network of FIG. 6, a portion of the ECG measurement nodes 100 are connected to the central access point 40 via fiber optic link 210, and another portion of the ECG measurement nodes 100 are wirelessly coupled to the central access point 40, the patient monitor 30, or one of the other ECG measurement nodes 100.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

The above-described examples of the described subject matter can be implemented in any of numerous ways. For example, some aspects may be implemented using hardware, software or a combination thereof. When any aspect is implemented at least in part in software, the software code can be executed on any suitable processor or collection of processors, whether provided in a single device or computer or distributed among multiple devices/computers.

The present disclosure may be implemented as a system, a method, and/or a computer program product at any possible technical detail level of integration. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some examples, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions may be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant may be entitled.

While various examples have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the examples described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific examples described herein. It is, therefore, to be understood that the foregoing examples are presented by way of example only and that, within the scope of the appended claims and equivalents thereto, examples may be practiced otherwise than as specifically described and claimed. Examples of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

## Claims

1. An electrocardiogram (ECG) measurement node (100) for a patient electrode (10), comprising:
an electrode interface (102) electrically and removably coupled to the patient electrode (10); and
ECG processing circuitry (104) electrically coupled to the electrode interface (102), wherein the ECG processing circuitry (104) is configured to generate an ECG signal (106).

2. The ECG measurement node (100) of claim 1, further comprising a battery (108).

3. The ECG measurement node (100) of claim 1, wherein the ECG processing circuit (104) comprises one or more amplifiers (110), one or more filters (112), and/or one or more analog-to-digital converters (118).

4. The ECG measurement node (100) of claim 1, further comprising a transceiver (114) configured to wirelessly transmit the ECG signal (106) and/or a control signal (122).

5. The ECG measurement node of claim 1, wherein the patient electrode (10) is affixed to a patient (20).

6. The ECG measurement node (100) of claim 1, further comprising a fiber optic interface (116) configured to transmit the ECG signal (106) and/or a control signal (122) via a fiber optic link (210).

7. An ECG measurement network (200), comprising:
a first ECG measurement node (100a) for a first patient electrode (10a), comprising:
a first electrode interface (102a) electrically and removably coupled to the first patient electrode (10a); and
first ECG processing circuitry (104a) electrically coupled to the first electrode interface (102a), wherein the first ECG processing circuitry (104a) is configured to generate a first ECG signal (106a); and
a second ECG measurement node (100b) for a second patient electrode (10b), comprising:
a second electrode interface (102b) electrically and removably coupled to the second patient electrode (10b); and
second ECG processing circuitry (104b) electrically coupled to the second electrode interface (102b), wherein the second ECG processing circuitry (104b) is configured to generate a second ECG signal (106b);
wherein the first ECG measurement node (100a) and the second ECG measurement node (100b) are communicatively coupled to a patient monitor (30).

8. The ECG measurement network (200) of claim 7, further comprising a central access point (40) communicatively coupled to the first ECG measurement node (100a), the second ECG measurement node (100b), and the patient monitor (30).

9. The ECG measurement network (200) of claim 8, wherein the central access point (40) is wirelessly coupled to the patient monitor (30).

10. The ECG measurement network (200) of claim 8, wherein the first ECG measurement node (100a) is communicatively coupled to the central access point (40) via a fiber optic link (210).

11. The ECG measurement network (200) of claim 7, wherein the first ECG measurement node (100a) is wirelessly coupled to the patient monitor (30).

12. The ECG measurement network (200) of claim 7, wherein the first ECG measurement node (100a) is communicatively coupled to the second ECG measurement node (100b).

13. The ECG measurement network (200) of claim 12, wherein the first ECG measurement node (100a) is wirelessly coupled to the second ECG measurement node (100b).

14. The ECG measurement network (200) of claim 12, wherein the first ECG measurement node (100a) is communicatively coupled to the second ECG measurement node (100b) via a central access point (40).

15. The ECG measurement network (200) of claim 12, wherein the first ECG measurement node (100a) and the second ECG measurement node (100b) form a local mesh network (250).
